# EUROPEAN PATENT APPLICATION

(11) **EP 4 645 332 A1**
(43) Date of publication of application: **05.11.2025**
(21) Application number: 25169557.3
(22) Date of filing: 09.04.2025
(51) Int. Cl.: G16H 30/20, G16H 30/40, G16H 40/63

(54) **SYSTEMS AND METHODS FOR DISPLAY OF MULTI-DATE AND MULTI-MODALITY IMAGES**

(30) Priority: 02.05.2024 US 202418653763
(71) Applicant: GE Precision Healthcare LLC, Waukesha, WI 53188 (US)
(72) Inventor: AMICE, Romane, 78530 Buc (FR); SPERANDIO, Melodie, 78530 Buc (FR); BAHUON, Adeline, 78530 Buc (FR); LOTH, Marine, 78530 Buc (FR); GUENNOUR, Marwa, 78530 Buc (FR); MONAHAN, Shannon, 78530 Buc (FR)
(74) Representative: Kilburn & Strode LLP

(57) **Abstract**

Methods and systems are herein provided for multi-date, multi-modality imaging display. In one example, a method for displaying multiple images in a graphical user interface (GUI) comprises obtaining one or more medical images and findings data thereof from a database (902); time ordering the obtained one or more medical images and findings data thereof (904); and displaying the one or more medical images and at least one corresponding findings data in respective viewports of the GUI based on a selected GUI configuration, wherein the one or more medical images are comparable images (910).

## Description

### FIELD

Embodiments of the subject matter disclosed herein relate to medical imaging displays, and more specifically to display of multi-date and multi-modality follow up imaging displays.

### BACKGROUND

Medical images, often stored and transmitted as digital files in Digital Imaging and Communications in Medicine (DICOM) format, are displayed via display devices, often in graphical user interfaces of workstations or picture archiving and communication systems (PACS). Medical images, such as images of internal anatomy of a human subject (e.g., patient) acquired by computerized tomography (CT), magnetic resonance imaging (MRI), ultrasound, and the like, are used for diagnosis, monitoring, screening, and more by physicians and other care providers in medical settings. Physicians and other care providers use data of the medical images, including series number, patient orientation data, etc., often displayed as annotation overlays within the graphical user interface, to aid in evaluation and diagnosis.

### BRIEF DESCRIPTION

In one example, a computing device comprises a display screen, the computing device being configured to display on the display screen a plurality of image viewports within a multi-image graphical user interface (GUI), the plurality of image viewports displaying respective medical images of a patient, and additionally being configured to display within the GUI one or more findings viewports each displaying findings data corresponding to an image displayed within one of the plurality of image viewports, wherein the findings data of each respective medical image of the patient is obtained from a database while the database is in an un-launched state, and wherein the respective medical images are comparable images of one or more imaging modalities.

In another example, a method for displaying multiple images in a graphical user interface (GUI) comprises obtaining one or more medical images and findings data thereof from a database; time ordering the obtained one or more medical images and findings data thereof; and displaying the one or more medical images in respective image viewports and findings data of at least one of the one or more medical images in at least one findings viewport of the GUI, wherein display of the respective image viewports and the at least one findings viewport is based on a selected GUI configuration, wherein the one or more medical images are comparable images of one or more imaging modalities.

In another example, a system comprises one or more imaging systems configured to acquire medical imaging data of a patient, wherein the one or more imaging systems are coupled to a database configured to store the medical imaging data and to a computing device configured with instructions stored in non-transitory memory executable by a processor that, when executed, cause the processor to: display a graphical user interface (GUI) comprising one or more image viewports and one or more findings viewports, wherein each image viewport displays a medical image of the medical imaging data and each findings viewport displays findings data corresponding to a respective medical image displayed within one of the one or more image viewports, wherein the one or more image viewports and the one or more findings viewports are arranged within the GUI according to a first GUI configuration; display one or more findings elements within each of the one or more findings viewports, wherein each of the one or more findings elements corresponds to an object of the medical imaging data, wherein the one or more findings elements display information relating to the object including percent change in volume of the object; and modify the GUI in response to user input to change from the first GUI configuration to a second GUI configuration, wherein in the first GUI configuration a set of comparable images are displayed in respective image viewports and in the second GUI configuration a subset of the set of comparable images are displayed in respective image viewports.

It should be understood that the brief description above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

The present invention will be better understood from reading the following description of non-limiting embodiments, with reference to the attached drawings, wherein below:
FIG. 1 shows a block diagram of an example computing system;
FIG. 2 shows an example of a graphical user interface (GUI) in a first configuration;
FIG. 3 shows an example of a GUI in a second configuration;
FIG. 4 shows a second example of the GUI of FIG. 3;
FIG. 5 shows an example of a GUI in a third configuration;
FIG. 6 shows a second example of the GUI of FIG. 5;
FIG. 7 shows a third example of the GUI of FIG. 5;
FIG. 8 shows a fourth example of the GUI of FIG. 5;
FIG. 9 shows a flowchart illustrating a method for displaying a multi-image GUI; and
FIG. 10 shows a flowchart illustrating a method for modifying display of a multi-image GUI.

### DETAILED DESCRIPTION

The following description relates to various embodiments for follow up medical imaging display. In particular, systems and methods for generating and displaying multi-date and multi-modality imaging studies within a single GUI for follow up comparison are provided. One or more images may be displayed within a GUI in a chosen configuration based on how a user prefers to interact with the presented data.

Hospitals and other clinical facilities may provide computing systems with graphical user interfaces (GUIs) for displaying patient medical images to care providers and other users. Medical images may be displayed on display devices (e.g., screens) of care provider devices in a suitable format such as Digital Imaging and Communications in Medicine (DICOM). Chronic diseases, such as cancer and neurodegenerative pathologies, demand long-term follow up, including repeated or serial imaging studies over time. Healthcare professionals assess evolution of disease and/or treatment response over months/years, for example evolution of a size of a tumor over time, in some examples in response to various treatments and interventions such as medications, surgical resection, radiation therapy, and chemotherapy. Assessment over time informs medical decision making and patient care. In order for proper assessment, an overview and a precise comparison between imaging studies, both a 1:1 comparison, for example between a most current and a previous study or between a most current and a first performed study, and multi-study comparison over a longer period of time, is used.

However, display of multiple images at the same time, so as to allow for efficient and accurate comparison, is cumbersome and time consuming. Further, multiple types of imaging may be used for disease assessment, for example computed tomography (CT), magnetic resonance imaging (MRI), positron emission tomography (PET), x-ray, ultrasound, mammography, and more, all of which may be presented separately in some systems. A care provider may have to find and open multiple studies individually, manually adjust window sizes to fit within a defined screen size, and scroll through slices of 3D images separately.

As an example, a patient being monitored for colon cancer who has had numerous types of studies over multiple months or years may undergo an abdominal CT following medical intervention (e.g., surgical excision, chemotherapy, etc.). A care provider treating the patient, in order to assess current disease status, may open the abdominal CT and separately open a most recent previous CT, a most recent previous PET scan, and a first performed abdominal CT taken prior to medical intervention. Each of the opened images may be opened in separate windows independent of one another. The care provider may then have to individually resize each of the separate windows as they desire. For example, the care provider may size the abdominal CT to half of a size of the screen of the care provider device and the first performed abdominal CT to a second half of the size of the screen so as to compare the two studies 1:1 to compare, for example, initial size of a tumor to a current size of a tumor. Then, the care provider may have to separately resize another one of the open images to allow for comparison between the three studies. The care provider may also have to manually scroll through image slices to allow for side by side comparison of similar cuts (e.g., to assess tumor size at the same coordinate over time). All in all, such image window opening, resizing, and manual comparison between images takes an undesirable amount of manual clicking by the care provider, thereby increasing overall time spent and reducing efficiency. Further, separately opening and interacting with individual studies may increase overall demanded processing power by the system, thereby decreasing efficiency of the computing device.

The methods and systems provided herein detail GUIs that display multiple medical images in various configurations. The GUIs herein described may comprise one or more image viewports each displaying a medical image therein. The medical images in each of the viewports may be interacted with individually and/or together, as will be herein described. The GUIs may also comprise one or more findings viewports that display information corresponding to one or more of the displayed images. In a first GUI configuration, the image viewports may be configured as a grid and one findings viewport may be displayed. The displayed findings viewport may correspond to a selected image of the displayed images. In a second GUI configuration, the image viewports may be configured horizontally and corresponding findings viewports for each of the display images may be configured horizontally, for example below the displayed image viewports. The imaging viewports and findings viewports may be arranged in a chronological, longitudinal manner. In a third GUI configuration, two image viewports may be arranged side by side to allow for a 1:1 comparison. Corresponding findings viewports for the two displayed images may also be displayed within the GUI in the third configuration. A user may toggle between each of the available GUI configurations based on desired view, clinical application, and/or intended use. Further, in the third, comparison configuration, the user may toggle between displayed images and findings in a chronological manner or may pin a chosen study to compare the chosen study to different other studies. Additionally, studies may be grouped within the third GUI, for example studies that were performed at substantially the same clinical time, and grouped images may be displayed alongside another single image for comparison therebetween.

The GUIs as herein presented may allow the user to visualize all or some comparable imaging studies within the same window. In some examples, the window size may be linked to an aspect ratio of the window in which the GUI. As such, each of the displayed imaging viewports may be displayed as the same size as one another, allowing for comparable views of anatomy within the displayed images. Further, the user may select a particular finding displayed within a findings viewport and all displayed images may show the particular finding at the same time (e.g., via comparable image slices). Thus, the GUIs as herein presented reduce time spent by the user in opening individual images, resizing image windows, and scrolling through image slices.

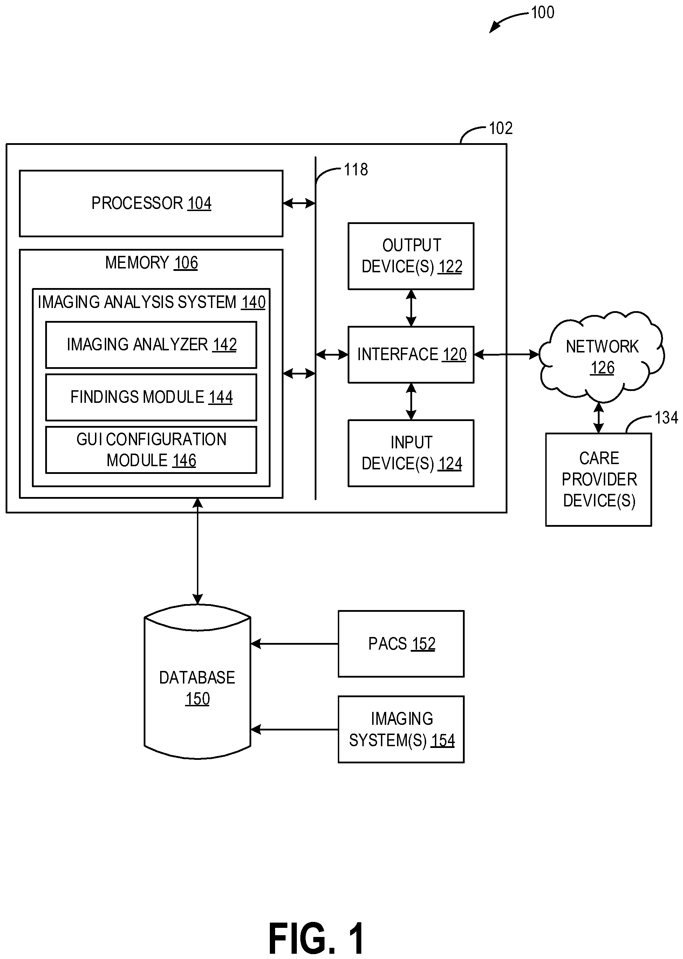

Referring now to FIG. 1, an example of a computing system 100 is shown. Computing system 100 comprises a computing device 102 which may comprise, as illustrative and non-limiting examples, a server, a personal computer, a workstation, a mobile device (e.g., a cellular phone, a smart phone, a computing tablet, and so on), or any other type of computing device.

The computing device 102 includes a processor 104 which may be configured to execute machine-readable instructions stored in memory 106. Processor 104 may be single core or multi-core, and the programs executed thereon may be configured for parallel or distributed processing. In some embodiments, the processor 104 may optionally include individual hardware components that are distributed throughout two or more devices, which may be remotely located and/or configured for coordinate processing. In some embodiments, one or more aspects of the processor 104 may be virtualized and executed by remotely-accessible networked computing devices configured in a cloud computing configuration.

As noted, the computing device 102 further includes memory 106. Memory 106 may include non-transitory memory, volatile memory, mass storage, local memory, the like, or some combination thereof. In some examples, memory 106 may include components disposed at two or more devices, which may be remotely located and/or configured for coordinate processing. In some embodiments, one or more aspects of the memory 106 may include remotely-accessible networked storage devices configured in a cloud computing configuration. The processor 104 and the memory 106 may be coupled, for example, via a communications bus 118.

The computing device 102 may further include an interface 120 communicatively coupled to the processor 104 and the memory 106 via the communications bus 118. The interface 120 may be implemented by one or more of any type of interface standard, such as an Ethernet interface, a universal serial bus (USB), a BLUETOOTH interface, a near field communication (NFC), and/or a PCI express interface.

The computing device 102 may further include one or more output device(s) 122 communicatively coupled to the processor 104 and the memory 106 via the interface 120. The output device(s) 122 may comprise, for example, one or more display devices. Such a display device may include one or more display devices utilizing virtually any type of technology (e.g., a light emitting diode (LED), an organic light emitting diode (OLED), a liquid crystal display (LCD), a cathode ray tube (CRT) display, an in-place switching (IPS) display, a touchscreen, and so on). In some examples, output device 122 may comprise a computer monitor configured to display medical information of various types and styles, including medical images. Output device(s) 122 may be combined with processor 104, memory 106, and/or user input device(s) 124 in a shared enclosure, or may be a peripheral display device and may comprise a monitor, touchscreen, projector, or other output device known in the art, which may enable a user to view decision support output (e.g., alerts) according to one or more examples of the current disclosure, and/or interact with various data stored in memory 106.

The computing device 102 may further include one or more user input device(s) 124 coupled to the processor 104 and the memory 106 via the interface 120. A user input device 124 may comprise, for example, one or more of a touchscreen, a keyboard, a mouse, a trackpad, a motion sensing camera, a microphone, or other device configured to enable a user to interact with and manipulate data within computing device 102.

The interface 120 may further include a communication device such as a transmitter, a receiver, a transceiver, a modem, a residential gateway, a wireless access point, and/or a network interface to facilitate exchange of data with external machines (e.g., computing devices of any kind) via a network 126. For example, the communication may be via, for example, an Ethernet connection, a digital subscriber line (DSL) connection, a telephone line connection, a coaxial cable system, a satellite system, a line-of-site wireless system, a cellular telephone system, and so on. As a non-limiting example, FIG. 1 shows one or more care provider devices 134 that may be communicatively coupled to computing device 102. Each care provider device may include a processor, memory, communication module, user input device, display (e.g., screen or monitor), and/or other subsystems (similar to the process, memory, communication module, user input device, and output device of computing device 102) and may be in the form of a desktop computing device, a laptop computing device, a tablet, a smart phone, or other device. Each care provider device may be adapted to send and receive encrypted data and display medical information, including medical images, in a suitable format such as DICOM or other standards. As will be explained in greater detail below, the care provider devices may display GUIs described herein on respective display screens.

An imaging analysis system 140 may obtain, analyze, and display one or more medical images of one or more patients based on instructions stored in memory 106. The imaging analysis system 140 may comprise an imaging analyzer 142, a findings module 144, and a GUI configuration module 146. The imaging analysis system 140 may be communicatively coupled to a database 150. The database 150 may store medical information, including medical images and data thereof, obtained from a picture archiving and communications system (PACS) 152 and/or one or more imaging system(s) 154. The imaging system(s) 154, such as CT systems, MRI systems, PET systems, and the like, may comprise an imager configured to acquire medical imaging data and may be configured to reconstruct medical images from the medical imaging data. The medical images may be stored via the PACS 152 or stored directly via the database 150.

The imaging analyzer 142 may analyze obtained medical imaging data and determine one or more parameters of corresponding medical images. In some examples, the imaging analyzer 142 may organize medical images by patient, date, type of scan, imaging protocol, and the like. The imaging analyzer 142 may obtain, generate, or otherwise determine dates of images, image series, patient orientation for images, and more. The imaging analyzer 142 may also include instructions for generating one or more reformations and/or renderings from the obtained medical images, such as multiplanar reformations (MPRs), maximum intensity projections (MIPs), and the like. Resulting reformations and/or renderings may be displayed in a GUI via the care provider device(s) 134. The imaging analyzer 142 may further generate and display annotation data, such as image series, date, and the like, for respective image data and image slices thereof, within image viewports. The imaging analyzer 142 may thus define comparable studies, wherein comparable studies include data of similar regions of the body, similar anatomy, etc., based on imaging protocol (e.g., scan range, protocol name, etc.).

The findings module 144 may obtain and/or generate data of one or more findings in each of the obtained imaging studies. For example, the findings module 144 may obtain and/or generate data of a size of a lesion at various slice locations based on generated segmentation data. The findings module 144 may also display the obtained and/or generated findings data within a GUI based on a current GUI configuration. For example, the findings module 144 may display findings viewports for each of the displayed image viewports in a horizontal configuration. The configuration of the GUI may be determined and generated by the GUI configuration module 146.

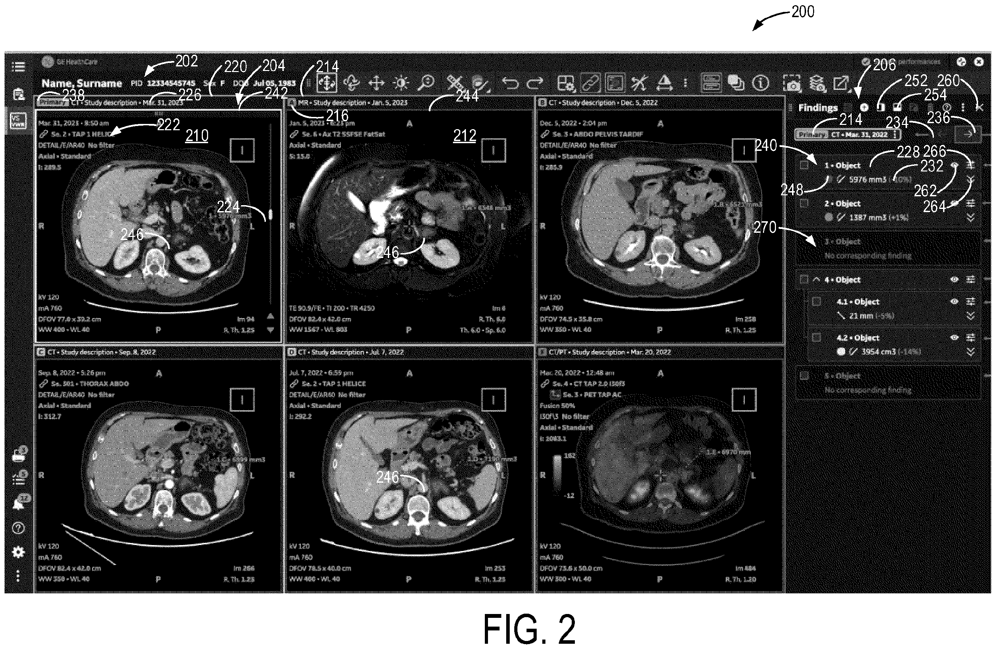

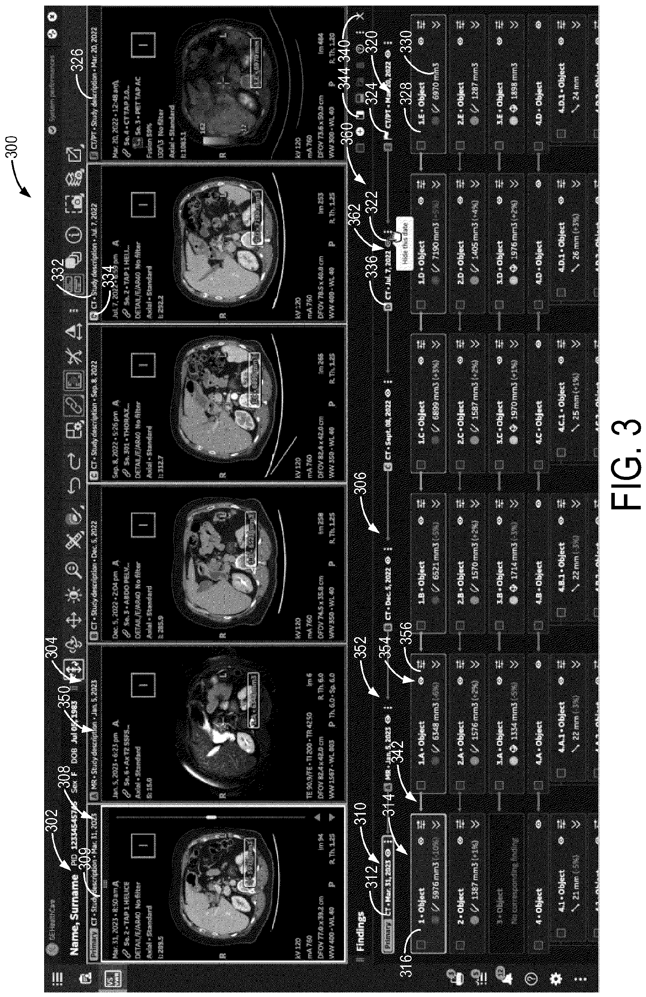

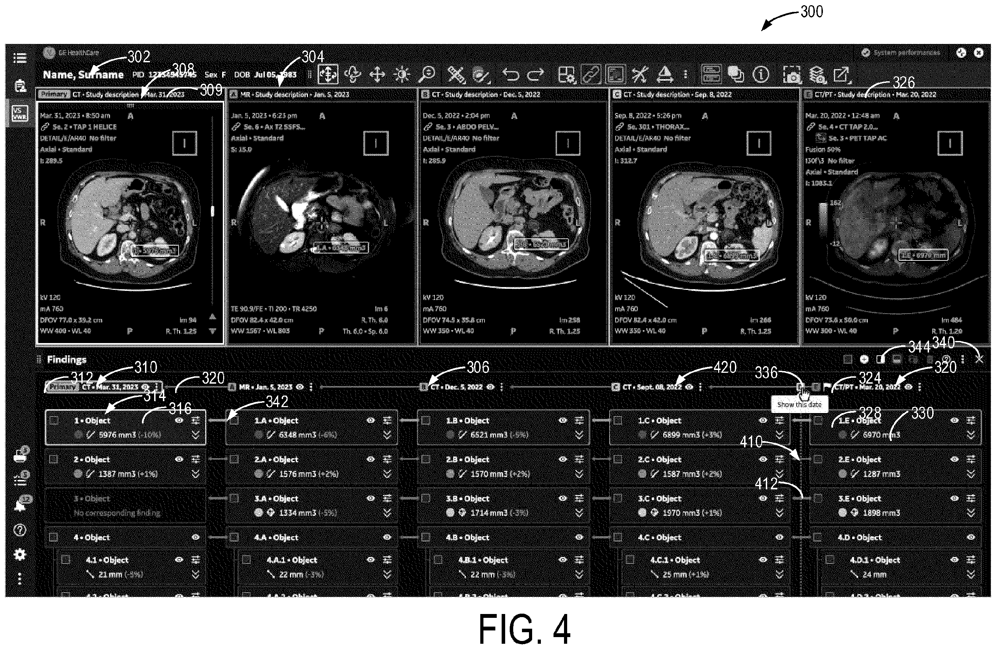

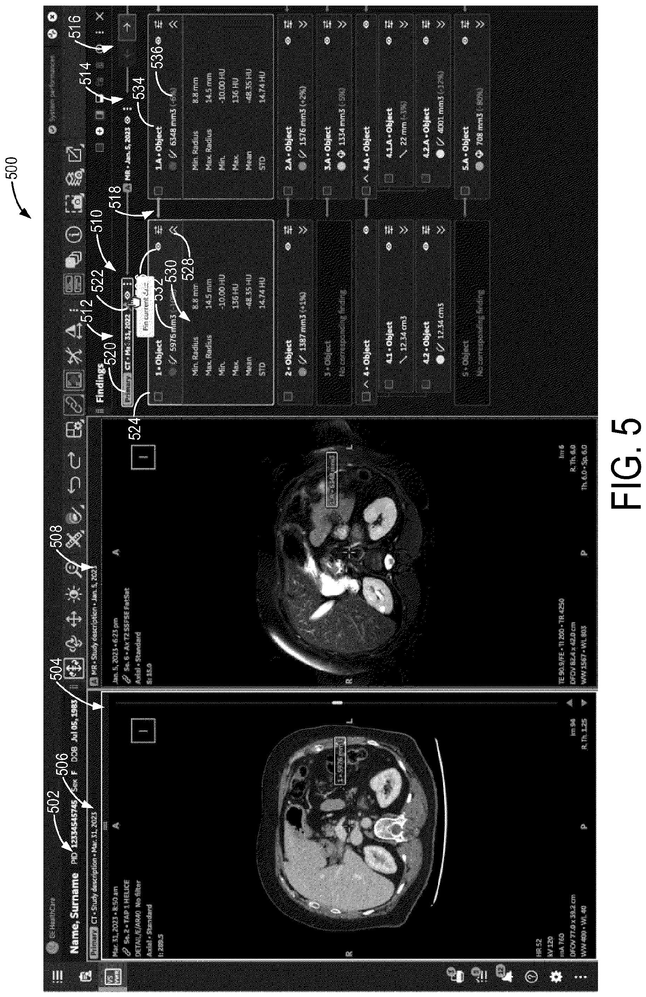

The GUI configuration module 146 may determine position and size of image viewports and findings viewports based on a selected GUI configuration. As previously described, the GUI may be displayed in a grid configuration, a horizontal configuration, or a comparison configuration. In the grid configuration, one or more image viewports are displayed in a grid and one findings viewport is displayed as a side panel, as will be described with respect to FIG. 2. In the horizontal configuration, comparable images are displayed horizontally in chronological order in image viewports and findings viewports are displayed for each of the displayed image viewports, as will be described with respect to FIGS. 3 and 4. In the comparison configuration, two images are displayed side by side in image viewports and corresponding findings viewports for the two displayed images are displayed side by side in a side panel, as will be explained with respect to FIGS. 5-8. The GUI configuration module 146 may also determine a selected image, whether images have been grouped, and whether an image has been pinned. Further, the GUI configuration module 146 may arrange findings viewports in order to align comparable findings elements that correspond to different displayed images. The GUI configuration module 146 may further determine relative sizing of the various image and findings viewports based on GUI window size, number of viewports, selected GUI configuration, and the like. The GUI configuration module 146 may thus automatically adjust viewport size and dimensions in response to changes in GUI window size based on an aspect ratio of the GUI window. The sizes of the displayed images may thus remain proportional and similar throughout despite changes in GUI window size. Further, as the GUI configuration module 146 adapts sizing of viewports automatically, the user need not do so manually, saving time and increasing efficiency.

Turning now to FIG. 2, an example first GUI 200 is shown. The first GUI 200 may be displayed on a care provider device, in some examples in response to user selection of a link within a menu and in other examples in a web browser window. The first GUI 200 is shown in a grid configuration. In some examples, the grid configuration may be a default GUI configuration. The first GUI 200 may display one or more image viewports 204 displaying a set of comparable images and a findings viewport 206 therewithin. Each of the images may correspond to a specified patient. The first GUI 200 may also comprise a patient demographic heading 202 that includes information such as patient name, patient birth date, patient medical identification number, patient sex, and the like. Further, the first GUI 200 may comprise a close element 260 for the findings viewport 206 that when selected may remove the findings viewport 206 from display. Configuration of the first GUI 200 may be manually changed from the grid configuration to the comparison configuration via selection of a comparison configuration element 252 or to the horizontal configuration via selection of a horizontal configuration element 254.

Each of the one or more image viewports 204 may display an image therewithin. In some examples, at any point in time, an image displayed may be an image slice of an imaging data set, when the image data set is a 3D data set. In other examples, an image displayed may be one of a plurality of 2D views of an imaging data set (e.g., in the case of multi-view x-ray imaging studies). Further, each of the image viewports may display annotation overlays therewithin, identifying data of the imaging study such as acquisition date, series, imaging sequence, current view (e.g., axial vs sagittal vs coronal) and the like. For example, a first image viewport 210 that displays a first image may comprise an annotation overlay 222.

Each of the image viewports may have a title line, an identifying outline, and an identifying icon. As an example, the first image viewport 210 may comprise a title line 226, which comprises a title of the imaging study therein displayed, a date the imaging study was acquired, and a study description, a first identifying outline 220, and a first identifying icon 238. In some examples, identifying outlines and identifying icons for each image viewport may be displayed with matching colors, thereby identifying the imaging study by the displayed color. For example, the first identifying outline 220 and the first identifying icon 238 may be displayed with a first color such as green. A second image viewport 212 displaying a second image may include a second identifying outline 214 and a second identifying icon 216 which may both be displayed with a second color, different from the first color, such as orange. Additionally, in some examples, the first identifying icon 238, when the first image viewport 210 is a "current" (e.g., a most recently acquired) imaging study displayed within the first GUI 200, may be a first type of icon, such as a teardrop that identifies the image of the first image viewport 210 as the current study. The other identifying icons for the other imaging viewports may be a second type of icons, such as a square with a letter therein. The displayed letters may correspond to time order of imaging studies, with a second most recent study being labeled "A", a third most recent study being labeled "B", and so on.

Further, each of the one or more image viewports 204 may be selectable. In the grid configuration as displayed, the findings viewport 206 may correspond to a selected image viewport. For example, the first image viewport 210 may be a selected image viewport of the one or more image viewports 204. As such, the findings viewport 206 may correspond to the first image viewport 210. Further, each of the one or more image viewports 204 may comprise a second outline that indicates whether the viewport is the selected viewport. For example, the first image viewport 210 may comprise a highlighted second outline 242 while the second image viewport 212 may comprise a non-highlighted second outline 244 indicating that the first image viewport 210 is selected and the second image viewport 212 is not selected. Additionally, the selected image viewport, in this case the first image viewport 210, may display a scroll bar 224 via which the user may scroll through image slices manually. In some examples, scrolling through image slices of the selected image viewport may also cause the other displayed images to match a current image slice (or most comparable image slice). In this way, scrolling through image slices of the selected study may amount to scrolling through selected slices of all displayed images. In other examples, scrolling through the selected image slices may be performed independent of the other displayed images.

The findings viewport 206, in the first GUI configuration, may comprise an image study heading 214 that includes identifying information for the corresponding imaging study, in this case the imaging study of the first image viewport 210. The findings viewport 206 further comprises one or more findings elements 240. Each of the findings elements 240 may correspond to an object, such as a lesion, tumor, area of inflammation, etc., which is seen within the corresponding imaging study. For example, a first findings element 228 may be displayed within the findings viewport 206. The first findings element 228, and other findings element therein displayed, may comprise various types of information relating to a corresponding object 246 as well as one or more selectable elements. For example, the first findings element 228 may comprise image series information, image slice information, and condensed object information, such as information of one or more metrics such as size or standardized uptake value (SUV). The information may include the metric itself as well as a percent change compared to a reference image. The condensed object information, as a non-limiting example, may indicate size of the object, including a total volume of the object 246 as well as a percentage change compared to a reference imaging study.

As an example, the first image may be a current study and the second image may be a most recent previous study to the first image (e.g., a longitudinally adjacent study). When the object information includes size information, as shown in FIG. 2, a condensed object size 232 of the first findings element 228 may indicate a total volume and a percentage change of the object 246 compared a reference image. It should be understood however that for different types of objects, other object information metrics may be displayed within In some examples, the condensed object size 232 as displayed within the first findings element 228 may be color coded based on the percentage change. For example, negative percentage changes (e.g., a statistically significant decrease in object size), may be displayed as green, neutral percentage changes (e.g., no statistically significant change) may be displayed as yellow, and positive percentage changes (e.g., a statistically significant increase in object size) may be displayed as red. In this way, at a glance, the user may easily determine change in a given metric (e.g., size of lesion, SUV of lesion, etc.). For example, for a size metric, the user may easily determine whether a lesion or other type of object is increasing in size or decreasing in size and by how much, thereby reducing time spent by the user in finding matching images and individually measuring lesion size.

The condensed object size 232 may have a corresponding object color 248 also displayed within the first findings element 228. The color of the object color 248 may correspond to a displayed color of the object within the one or more image viewports 204 when the first findings element 228 is selected. For example, when the first findings element 228 is selected, the object 246 to which it corresponds may be highlighted in the color of the object color 248 within one or more of the one or more image viewports 204. The one or more of the one or more image viewports 204 that display the highlighted object 246 may correspond to the images in which the object 246 is present.

As noted, each findings element may comprise one or more selectable elements. For example, within the first findings element 228, the one or more selectable elements may comprise a hide element 262, an expansion element 264, and an additional information element 266. The hide element 262, when selected may remove the first findings element 228 from display within the findings viewport 206. The expansion element 264, when selected, may trigger display of additional information of the object 246, including minimum radius, maximum radius, minimum radio density (e.g., in Hounsfield units (HUs)), maximum radio density, and the like.

In some examples, an object may be present in one image but not present in another, for example following surgical resection or chemotherapy treatment. In such examples, a corresponding findings element may indicate that no corresponding finding is found. For example, a second findings element 270 of the findings viewport 206 includes indication of no corresponding finding in place of a percentage change amount. Further, the second findings element 270 may be shaded, indicating the lack of the object to which the findings element would correspond in the corresponding image.

The selected image viewport may be manually changed via user selection of another image viewport. Alternatively, the selected image viewport may be changed via user selection of a next element 234 or a previous element 236. The next element 234, when selected, may select a next image viewport based on date of acquisition of a corresponding image (e.g., a next later acquired image). The previous element 236, when selected may select a previous image viewport based on date of acquisition of a corresponding image (e.g., a next earlier acquired image). Consequently, the findings viewport 206 may also update to display information relating to the newly selected image viewport. As an example, selection of the previous element 236 may deselect the first image viewport 210 and may select the second image viewport 212. The findings viewport 206 may thus display information relating to the second image displayed within the second image viewport 212. In this way, information relating to multiple images acquired across a variety of dates and with a variety of modalities may be viewed within the same GUI.

The one or more image viewports 204 in the grid configuration may be configured with a shape and size to fit within a window of the first GUI 200. Further, the shape and size of the viewports may depend on how many comparable studies are available for display. For example, six image viewports are displayed within the first GUI 200. However, if four comparable images are available, a size of the viewports may be larger than when six are available. Further, one or more viewports may be larger than other viewports included in the grid in examples in which the number of available viewports is too small to fill every viewport in the assigned grid size. The arrangement of images within the image viewports may be left to right in decreasing time order for each row, in some examples, or from right to left, depending on user preference.

In this way, the first GUI 200 in the first configuration may allow the user to see each of the set of comparable studies at the same time without having to manually resize individual windows. This reduces time spent by the user in setting up viewports and also may reduce processing power used by the computing device by reducing the number of interactions the user makes with the viewports.

Turning now to FIG. 3, a second GUI 300 is shown. The second GUI 300, similar to the first GUI 200, may be displayed on a care provider device. The second GUI 300 may be in a horizontal configuration. In some examples, the second GUI 300 may be displayed in response to user selection of a horizontal configuration element within a GUI in another configuration. For example, the first GUI 200 as described above may be in the grid configuration and may comprise the horizontal configuration element 254. The horizontal configuration element 254, when selected, may launch the second GUI 300.

The second GUI 300 may include a patient demographic heading 302 that displays patient name, patient birth date, patient identification number, and patient sex. The second GUI 300 may further include one or more image viewports 304 and one or more findings viewports 306. Each of the one or more findings viewports 306 may correspond to and vertically align with one of the one or more image viewports 304. The second GUI 300, similar to the first GUI 200, may comprise a close element 340 for the one or more findings viewports 306 that when selected may remove the findings viewports 306 from display. Configuration of the second GUI 300 may be manually changed from the horizontal configuration to the comparison configuration via selection of a comparison configuration element 344 or to the grid configuration via selection of a grid configuration element.

As described with regards to FIG. 2, each of the one or more image viewports may display an image therein, may have a title, an identifying outline, and an identifying icon. Each of the one or more findings viewports may similarly comprise a title element that identifies which image study it corresponds to. The title element may also include the same identifying icon as the corresponding image viewports. For example, a first image viewport 308 may correspond to a first findings viewport 310. The first image viewport 308 may comprise a header 309 that comprises information such as study modality, study description, and acquisition date as well as the identifying icon. The first findings viewport 310 may comprise a corresponding header 312 that comprises information such as study modality and acquisition date as well as the identifying icon. The first image displayed within the first image viewport 308 may be a current image and thus the identifying icon thereof may be the current study icon (e.g., the teardrop pin icon), thereby visually indicating that the first image is the current study.

Similar to the first GUI 200, each of the findings viewports 306 may comprise one or more findings elements. For example, the first findings viewport 310 may comprise one or more findings elements 314, including first findings element 316, that each display information relating to an object, including a total volume of the object in the corresponding image. Each of one or more findings elements 314 may be linked to a findings element of an adjacent findings viewport. For example, a second image viewport 350 may be directly adjacent to the first image viewport 308 (e.g., from a longitudinal, chronological perspective as well as visually in some examples) and may correspond to a second findings viewport 352, which may be directly adjacent to the first findings viewport 310. The second findings viewport 352 may comprise one or more findings elements 354 each of which may align with the one or more findings elements 314. A plurality of linking arrows 342 may be arranged between the first findings elements 314 and the second findings elements 354. The plurality of linking arrows 342 may comprise a linking arrow between each of the aligned findings elements. The linking arrow may point in the direction of increasing acquisition dates. The linking arrows 342 may indicate visually to the user which object measurements are being compared in displayed percentage changes. For example, a linking arrow may extend from a second findings element 356 of the one or more findings element 354 of the second findings viewport 352 to the first findings element 316 of the first findings viewport 310. The first findings element 316 may include a percentage change amount, as previously described, and the percentage change amount may reference the percentage change compared to information of the object displayed in the linked second findings element 356.

As described with respect to FIG. 2, each of the image viewports 304 and each findings element of each of the findings viewports 306 may be selectable elements. The findings elements of the findings viewports 306 may be linked to a corresponding image viewport, whereby selection of a findings element also selects a corresponding image viewport. For example, the first findings element 316 of the first findings viewport 310 is selected and the first image viewport 308 may also be selected. Further, an object to which the first findings element 316 corresponds may be highlighted within each of the displayed image viewports (e.g., a segmentation mask may be overlaid on each displayed images).

The percentage change for the object may be displayed within each findings viewport except a findings viewport corresponding to a first acquired image, in some examples. For example, a fourth imaging viewport 326 may correspond to a fourth findings viewport 320. The fourth imaging viewport 326 may display a first acquired image of the comparable images displayed within the second GUI 300. Further, the fourth findings viewport 320 may comprise may comprise a flag icon 324 in a heading thereof. The flag icon 324 may indicate that the corresponding image is a reference image. In some examples, the reference image may be the first acquired image in which one or more objects were noted. For example, an imaging study may be performed for a symptom of a patient and the imaging study may show a lesion for the first time. This imaging study may thus become a reference study for all subsequently acquired comparable images. In other examples, the reference image may not be the first acquired image, depending on clinical application. For example, a pre-therapy imaging study may be designated as the reference study in order for comparison of pre-therapy to post-therapy to be made. The fourth findings viewport 320 may comprise a third findings element 328 that corresponds to the object of the first findings element 316 and second findings element 356. The third findings element 328 may not include a percentage change amount, but rather may indicate a baseline object metric 330 such as a baseline object size when the corresponding image is the reference image. As a point of comparison, in the grid configuration, the findings viewport may additionally include a flag icon when the reference image is selected.

Further, each of the headings of each of the one or more findings viewports 306 may comprise selectable elements including a hide element and an additional actions element. For example, a third image viewport 332 which has an identifying icon 334 may correspond to a third findings viewport 360 with a heading 362. The heading 362 may comprise an identifying icon 336 identical to the identifying icon 334 of the third image viewport 332. The heading 362 may additionally comprise a hide element 322. The hide element 322, when selected via a first user input such as a mouse click, may trigger removal of the third image viewport 332 and the third findings viewport 360 from display within the second GUI 300. The hide element 322 may also be selectable via another type of user input such as a hover that displays a pop-up window indicating a result of the first user input (e.g., indicating that the viewports will be hid upon selection of the hide element).

The one or more image viewports 304 may be arranged horizontally along a first portion of the second GUI 300 in a time ordered fashion. The one or more image viewports 304 may comprise all available comparable imaging studies for the patient. Comparable imaging studies may be imaging studies that include similar patient anatomy, in some examples. For example, the one or more image viewports 304 shown in FIG. 3 include images acquired of an abdomen. Comparable images may include images of various modalities, for example CT Abdomen, CT Abdomen/Pelvis, MRI Abdomen, and PET/CT Abdomen may all be considered comparable studies that image anatomy of the abdomen.

The one or more findings viewports 306 may be arranged horizontally along a second portion of the second GUI 300. As is shown in FIGS. 3 and 4, the first portion may be an upper portion and the second portion may be a lower portion arranged below the one or more image viewports 304. It should be understood however that the findings viewports may be arranged in an upper portion and the image viewports may be arranged in a lower portion, in other examples.

When a set of image and findings viewports are hidden, the second GUI 300 may automatically adapt to expand a size of each of the remaining image viewports and each of the remaining findings viewports to fit within the GUI window. In this way, the GUI may be configured to adapt window sizes of each of the viewports based on aspect ratio and size of the GUI window, the number of viewports, and the GUI configuration.

Turning to FIG. 4, the second GUI 300 is again shown. The second GUI 300 as shown in FIG. 4 may be displayed in response to user selection of the hide element 322 of FIG. 3, for example via the first user input (e.g., mouse click). The second GUI 300, following hiding of the third imaging and findings viewports, may display each of the remaining imaging and findings viewports in a similar fashion to prior to the hiding. As noted, the second GUI 300 may be configured to resize each of the remaining viewports to fit within the same GUI window size.

The second GUI 300, following hiding of the third imaging and findings viewports, may continue to display the identifying icon 336 thereof. The identifying icon 336, when the corresponding viewports are hidden, may be a selectable element that when selected via user input such as a mouse click may trigger display of the corresponding findings and image viewports (e.g., thereby returning the second GUI 300 to the view shown in FIG. 3). Further, with the third findings viewport hidden, linking arrows 410 may be displayed between a next findings viewport 420 and the previous findings viewport 320 (e.g., the fourth findings viewport 320) of the third image. Additionally, a dashed line 412 may be positioned between the next findings viewport and the previous findings viewport as a placeholder, indicating that an image exists therebetween.

In some examples, the percentage changes displayed within the next findings viewport 420 may relate to the previous findings viewport 320, skipping over the findings of the third findings viewport when the third findings viewport is hidden. In other examples, the percentage changes displayed within the next findings viewport 420 may relate to the hidden third findings viewport, the dashed line 412 indicating the existence of the hidden findings viewport.

Turning now to FIG. 5, a third GUI 500 is shown. The third GUI 500, similar to the first GUI 200 and second GUI 300, may be displayed on a care provider device. The third GUI 500 may be in a comparison configuration (e.g., vertical configuration). In some examples, the third GUI 500 may be displayed in response to user selection of a comparison configuration element within a GUI in another configuration. For example, the first GUI 200 as described above may be in the grid configuration and may comprise the comparison configuration element 252. The comparison configuration element 252, when selected, may launch the third GUI 500. The second GUI 300 may comprise a similar comparison configuration element (e.g., comparison configuration element 344) that also when selected may launch the third GUI 500, in some examples.

The third GUI 500 may include a patient demographic heading 502 that displays patient name, patient birth date, patient identification number, and patient sex. The third GUI 500 may further include two or more image viewports 504 and two or more findings viewports 510 corresponding to a subset of the set of comparable images. Each of the two or more findings viewports 510 may correspond with one of the two or more image viewports 504. As described with regards to FIG. 2, each of the two or more image viewports may display an image therein, may have a title, an identifying outline, and an identifying icon. Each of the two or more findings viewports may similarly comprise a title element that identifies which image study it corresponds to. The title element may also include the same identifying icon as the corresponding image viewports. For example, a first image viewport 506 may correspond to a first findings viewport 512. The first image viewport 506 may comprise a header that comprises information such as study modality, study description, and acquisition date as well as the identifying icon. The first findings viewport 512 may comprise a corresponding header 520 that comprises information such as study modality and acquisition date as well as the identifying icon.

Similar to the first GUI 200 and the second GUI 300, each of the findings viewports 510 may comprise one or more findings elements indicating data of corresponding objects, such as total volume of the object. For example, the first findings viewport 512 may comprise one or more findings elements, including first findings element 524. Each of one or more findings elements may be linked to a findings element of an adjacent findings viewport. For example, a second image viewport 508 may be adjacent to the first image viewport 506 and may correspond to a second findings viewport 514, which may be adjacent to the first findings viewport 512 (e.g., both visually and chronologically). The second findings viewport 514 may comprise one or more findings elements each of which may align with the one or more findings elements of the first findings viewport 512. A plurality of linking arrows 518 may be arranged between the first findings elements 512 and the second findings elements 514. The plurality of linking arrows 518 may comprise a linking arrow between each of the aligned findings elements. The linking arrow may point in the direction of increasing acquisition dates. The linking arrows 518 may indicate visually to the user which object measurements are being compared in displayed percentage changes.

For example, a linking arrow may extend from a second findings element 534 of the one or more findings element of the second findings viewport 514 to the first findings element 524 of the first findings viewport 512. The first findings element 524 may include a percentage change amount 532, as previously described, and the percentage change amount 532 may reference the percentage change in the given metric (e.g., object size, SUV, etc.) of the corresponding object in the first image compared to the second image to which the linked second findings element 534 corresponds. The second findings element 534 may also comprise a percentage change amount 536, which may be based on a comparison to a next most recent study of which findings elements are not currently displayed within the third GUI 500.

Further, each of the findings elements may be color coded, as described previously, and when selected a corresponding object may be highlighted with the corresponding color within each of the displayed images. Additionally, each of the findings elements may comprise one or more selectable elements. For example, the first findings element 524 comprises a hide element 526 that when selected removes the first findings element 524 from display within the first findings viewport 512, and an expansion element 528. The expansion element 528, when selected, as is shown in FIG. 4, may expand the first findings element 524 to display additional information 530 of the object to which the first findings element 524 corresponds, including minimum radius measurement, maximum radius measurement, minimum radio density, maximum radio density, mean radio density, and standard deviation of radio density. In some examples, expansion of the first findings element 524 may also trigger expansion of the second findings element 534 to which the first findings element 524 is linked. In this way, the user may directly compare one or more parameters of the object between different imaging studies without having to open multiple windows or individually interact with images.

The third GUI 500 may further comprise a displayed image change element 516 that comprise a next element and a previous element. When no image is pinned, the next element, when selected via user input, may trigger shifting of images and findings in viewports to display a next previously acquired image in comparison to the second image. Specifically, the image displayed within the second image viewport 508 may shift to being displayed within the first findings viewport 506 and the image displayed within the first findings viewport 506 to be removed from display. A third image may be displayed within the second image viewport 508, replacing the image that moves to the first findings viewport 506. Similarly, the information presented within the first findings viewport 512 may be removed from display, the information presented in the second findings viewport 514 may shift to being displayed within the first findings viewport 512, and information of the third image may be presented within the second findings viewport 514. In this way, the next element may allow for viewing of a subsequent image for 1:1 comparison with an adjacent image. The previous element may have a similar but opposite effect, whereby images and findings displayed in viewports may be shifted to display a more recently acquired image in comparison to the adjacent previously acquired image (e.g., the first image). Thus, the user may easily toggle between images to compare different images over time.

Further, the header 520 of the first findings viewport 512 may additionally comprise a pin element 522. The pin element 522, when selected via user input, may pin the imaging study current displayed within the first imaging and findings viewports 506, 512 to the third GUI 500. Thus, when the user changes which images and information thereof are displayed, for example via the displayed image change element 516, only the image in the second image viewport 508 and the information in the second findings viewport 514 may shift while the currently displayed image in the first image viewport 506 and the corresponding information displayed within the first findings viewport 512 may remain unchanged. In this way, the user may compare a pinned image study (e.g., the images thereof and the findings thereof) to each of the other available comparable images individually instead of only the adjacent imaging studies.

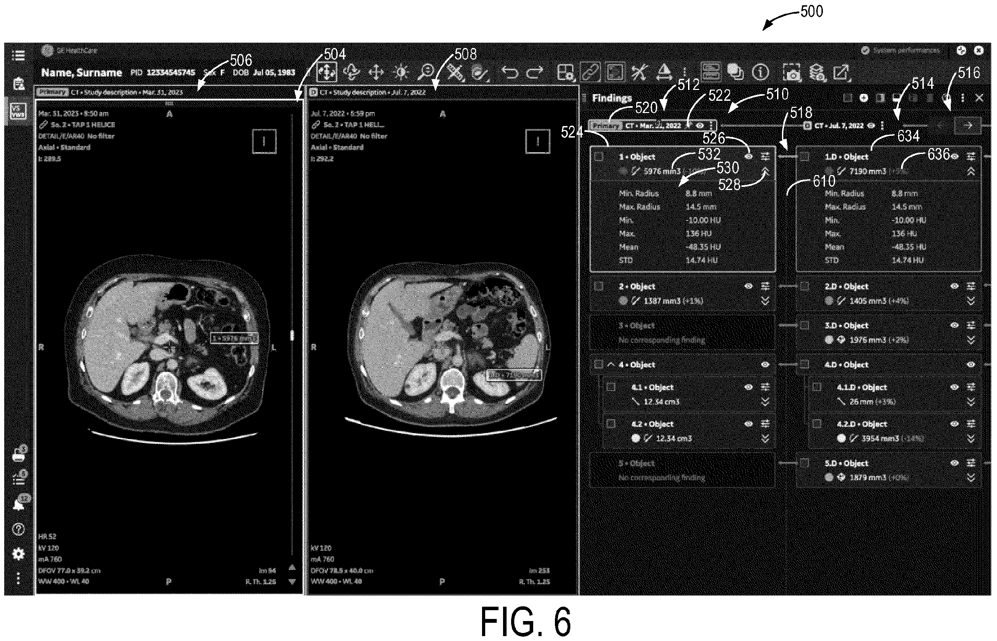

For example, FIG. 6 shows the third GUI 500 with the first imaging study pinned. While the third GUI 500 as shown in FIG. 5 shows the first image and the second images and findings thereof displayed in viewports, where the first and second images are chronologically adjacent to each other, the third GUI 500 as shown in FIG. 6 shows the first image and a third image displayed in viewports. The first image and the third image may not be chronologically adjacent to each other. With the first imaging study pinned, as shown in FIG. 6, the user may toggle display of different images in the second image viewport 508 via the displayed image change element 516. Without a pinned study, the displayed images may always be chronologically adjacent to each other. With a study pinned, as shown in FIG. 6, the displayed images may not necessarily be chronologically adjacent to each other.

For example, the first image viewport 506 may display the first image of the pinned study and the second image viewport 508 may display the third image. The first findings viewport 512 may remain unchanged compared to as described with respect to FIG. 5, in some examples. However, the second findings viewport 514, when the second image viewport 508 displays the third image in response to user selection of the third imaging study (e.g., via selection of the next element of the image change element 516 one or more times), may indicate findings of the third image. Findings elements of the second findings viewport 514 may remain linked to the findings elements of the first findings viewport 512, as previously described. For example, the second findings viewport 514 may include a third findings element 634 that indicates information 636, such as total volume and percent change in volume, of the object to which the first and third findings elements 524, 634 correspond.

When the displayed images and findings thereof are not chronologically adjacent, as depicted in FIG. 6 with the first study pinned, a separator line 610 may be displayed between the first and second findings viewports 512, 514. The separator line 610 may indicate that one or more imaging studies are available between the first and third images currently displayed. The linking arrows 518 may link findings elements as previously noted, however the separator line 610 may indicate to the user that the third image currently displayed is not longitudinally adjacent to the first image.

Pinning a study allows the user to directly compare a selected image and findings thereof to each of the other available comparable images individually as desired. For example, the pinned study may be a most recently acquired imaging study and the user may first compare one or more metrics, such as size and SUV, of an object in the first imaging study to a most recently acquired imaging study. Then, the user may toggle to display a second previously acquired imaging study and may compare the first imaging study to the second previously acquired imaging study. In this way, the user may be able to directly compare not only chronologically adjacent images but also non-chronologically adjacent images, allowing for comparison over larger periods of time within the same single GUI.

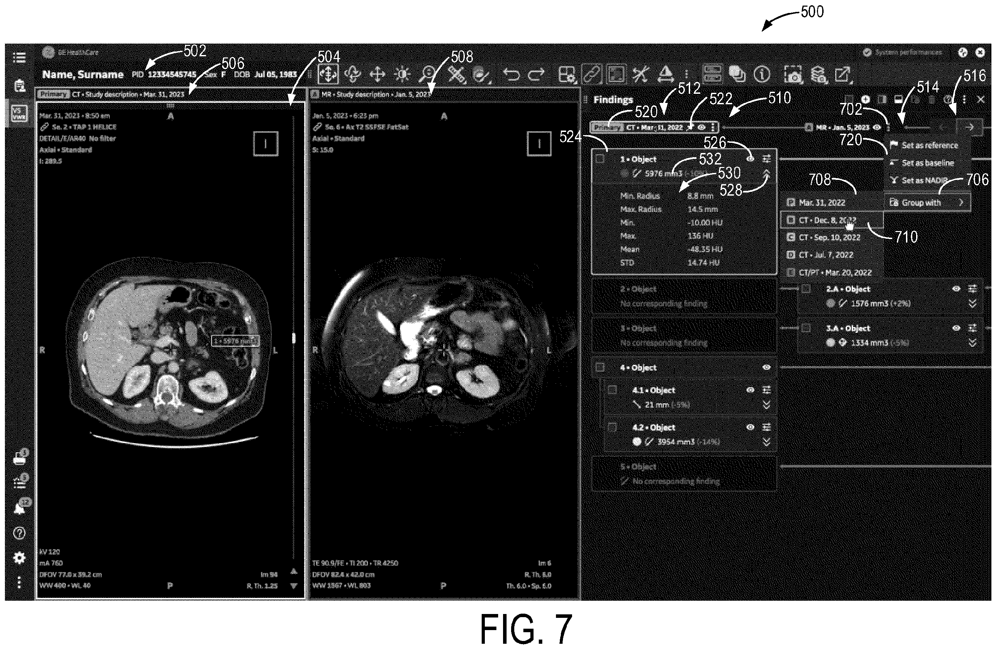

Turning now to FIG. 7, the third GUI 500 is again shown, similar to FIG. 5. The third GUI 500 as shown in FIG. 7 displays the first image in the first image viewport 506 and the second image in the second image viewport 508. The comparison GUI configuration may allow the user to group one or more imaging studies. For example, some imaging studies may be considered as occurring at the "same time" from a clinical point of view despite having different dates or timestamps. For example, a patient may undergo a CT for evaluation of a symptom at a first date and then based on findings thereof, may undergo an MRI for additional characterization of the findings. The CT and the MRI may be acquired on different dates, but due to a time period therebetween being clinically insignificant, without medical intervention therebetween, and/or without significant change in findings therebetween, the two studies may be considered together. For example, the CT and the MRI in the presented scenario may both be considered pre-intervention studies and thus a care provider may consider them as being acquired at the "same time" from a clinical standpoint.

In such a scenario, the user may group studies within the GUI. For example, the second image as displayed in the third GUI 500 may be grouped with a third image. As noted previously, the second findings viewport 514 may comprise a heading with an additional actions element 702. The additional actions element 702, when selected via user input, may trigger display of a first pop-up menu 720. The first pop-up menu 720 may comprise a plurality of selectable action elements. For example, the first pop-up menu 720 may include a selectable element that may set the second image as a reference image, a baseline image, and/or as a nadir image (e.g., an image of the available comparable images in which one or more objects are at their smallest). The pop-up menu 720 may also include a grouping element 706.

The grouping element 706, when selected may trigger display of a second pop-up menu 708. The second pop-up menu 708 may comprise a list of available imaging studies with which the second image may be grouped. Selecting one of the studies from the list may trigger the system to group the second study with the selected study. For example, user selection of listed study 710 may trigger grouping of the second study with the listed study 710 (e.g., the third image).

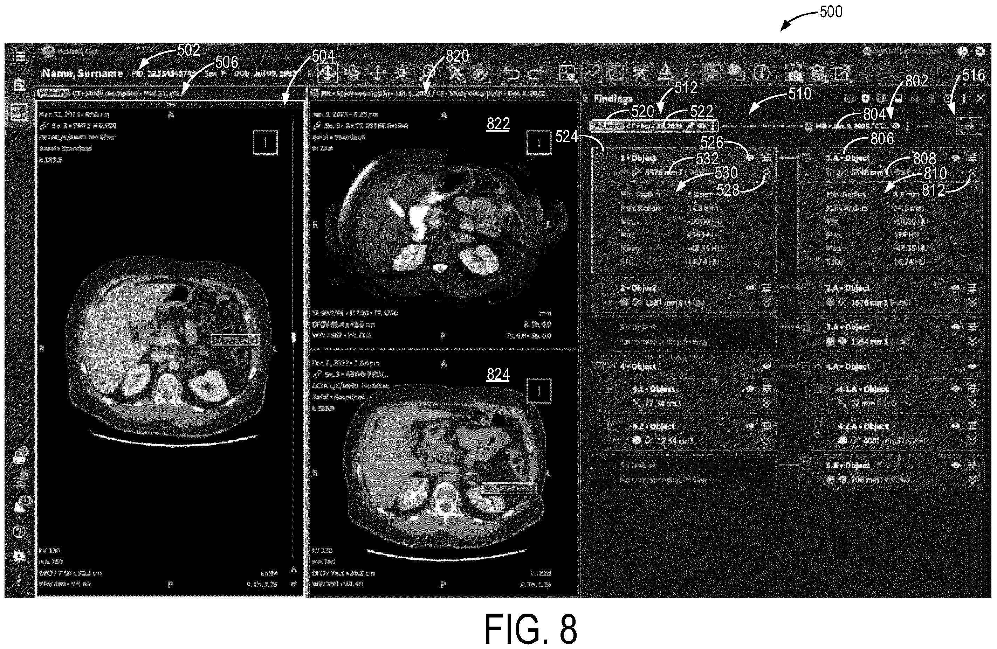

In response to grouping of studies as described above, the third GUI 500 may be displayed with grouped studies, as shown in FIG. 8. The third GUI 500 when including grouped studies, as shown in FIG. 8, may include the first image viewport 506 and a grouped image viewport 820. Similarly, the third GUI 500 when including grouped studies may include the first findings viewport 512 and a grouped findings viewport 802. The grouped image viewport 820 may be the second image viewport 508 with more than one image displayed therein, as will be further explained. The grouped findings viewport 802 may be the second findings viewport 514 with findings data of more than one image displayed therein, as will be further explained.

For example, the grouped image viewport 820 (e.g., the second image viewport 508 when studies are grouped) may comprise a first grouped image viewport 822 and a second grouped image viewport 824. As an example, the first grouped image viewport 822 may display the second image that was displayed within the second image viewport 508 and the second grouped image viewport 824 may display the third image of the listed study 710 that was grouped with the second image.

The grouped image viewports 822, 824 may be arranged within the third GUI 500 vertically so as to occupy the same space as the second image viewport 508. Thus, the GUI may configure the size of the grouped image viewports as well as the displayed annotations, displayed image, and the like therewithin. Each of the first and second grouped image viewports 822, 824 may be individually selectable and/or collectively selectable. When selected, the user may interact with the selected image viewport, including scrolling through image slices of the corresponding image, selecting on various elements such as annotation elements and the like.

The grouped findings viewport 802 may comprise a heading 804. The heading 804 may indicate study modality/protocol and date for each studies that are grouped, such as the second image and the third image. In some examples, the grouped studies may have the same or similar objects and object sizes as each other and thus data reported in one or more findings element within the grouped findings viewport 802 may be shared data.

As an example, the grouped findings viewport 802 may comprise a first grouped findings element 806. The first grouped findings element 806 may comprise data corresponding to the object of the first findings element 524, for example size information 808, including measured volume and percent change in volume. Other metrics, such as SUV may be included as well in some examples, depending on clinical case. In some examples, the first grouped findings element 806 may display size information for each of the grouped imaging studies separately, whether or not statistically significant differences in size exist therebetween.

Similar to other findings element described herein, the first grouped findings element 806 may comprise an expansion element 812 that when selected or triggered as selected (e.g., via user selection of the expansion element 528 of the first findings element 524 of the first findings viewport 512) triggers display of additional information 810 of the object. The additional information 810 may directly correspond to the additional information 530 of the first image to allow for comparison between the object in the first image and the object in the grouped images.

When the third GUI 500 includes grouped imaging studies, as shown in FIG. 8, the displayed image change element 516 may allow the user to toggle between displays of imaging studies while maintaining the grouping as selected by the user. For example, selection display of a previous study may trigger display of a fourth image alongside the grouped second and third images. Thus, grouping of images may group the grouped images, indicating to the system that the grouped images are to be considered as one study for display purposes.

When the third GUI 500 includes grouped imaging studies, as shown in FIG. 8, the user may toggle to a different GUI configuration, such as the grid configuration shown in FIG. 2 or the horizontal configuration shown in FIGS. 3-4. In some examples, when the user toggles to a different GUI configuration, the grouped studies may remain grouped in the display. In other examples, when the user toggles to a different GUI configuration, the grouped studies may be split and displayed individually. As an example, when toggling from the comparison configuration of the third GUI 500 to the horizontal configuration of the second GUI 300, the grouped studies may remain grouped and display of the horizontal configuration may be adjusted to accommodate display of two image viewports within a size of one of the other image viewports. In this way, the system as herein presented may accommodate various image viewport presentations based on user interaction. As another example, when toggling from the comparison configuration of the third GUI 500 to the grid configuration of the first GUI 200, the grouped studies may be split and displayed individually. When the grouped studies are displayed individually, such as in the grid configuration, the grouping may be displayed visually via a shared identifying icon and identifying border color. Splitting of grouped studies may not change the grouping of the studies within stored memory such that upon toggling back to the comparison configuration, the studies may again be displayed as grouped.

The GUI as presented thereby allows the user to selectably toggle between the various configurations of the GUI as herein presented based on desired use. For example, the comparison configuration may allow for direct comparison of two chosen images and corresponding findings while the horizontal configuration shown in FIGS. 3 and 4 may allow for a longitudinal comparison of images and findings over a longer period of time. Thus, as a non-limiting example, the horizontal configuration may allow the user to view change in size of a lesion from a first obtained image to a most recently obtained image while the comparison configuration may allow the user to view change in size of the lesion from the most recently obtained image to a specific chosen previously obtained image. Each may have clinical uses for different users and thus the system herein provided may be tailored to different use case scenarios.

Each of the available GUI configurations may be defined by a user as a default. For example, a radiologist, who may desire to focus on lesion sizes in directly compared images in order to indicate in their findings whether the lesion has increased in size, decreased in size, or remained stable compared to a most recent previous image, may select the comparison configuration as a default. An oncologist, however, who may desire to focus on overall evolution of images and findings such as lesion size in order to determine next course of treatment, may select the horizontal configuration as default. The system and GUIs herein provided may thus be flexible and adaptive to various user preferences.

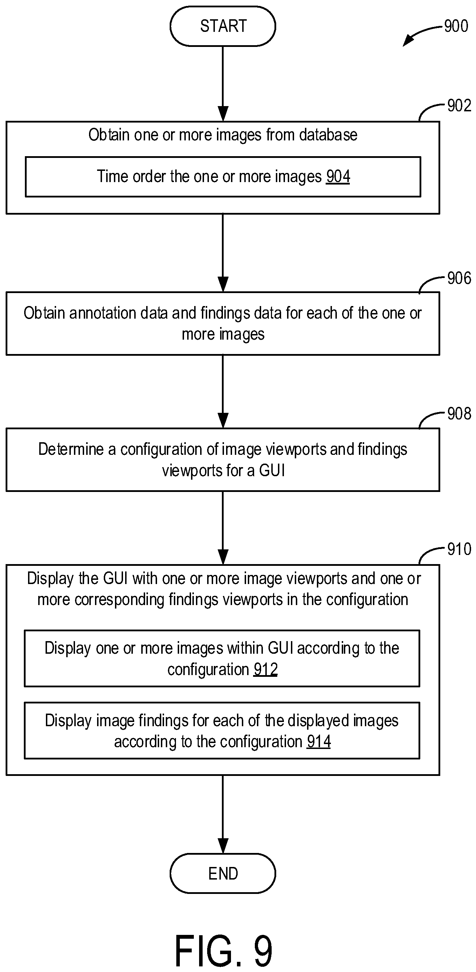

Turning now to FIG. 9, a flowchart illustrating a method 900 for displaying a multi-date imaging GUI is shown. The method 900 may be carried out using the systems and components described herein above with regards to FIG. 1. For example, the method 900 may be carried out according to instructions stored in memory of one or more processors of a computing device, such as computing device 102, which is communicatively coupled to a display device configured to display a GUI, such as the first GUI 200, the second GUI 300, and/or the third GUI 500.

At 902, method 900 includes obtaining one or more images from a database. The one or more images may be specific to a patient and may include image data of various types of renderings, such as MPRs and/or MIPs, and of various imaging modalities, such as CT, MRI, x-ray, PET, and the like. In some examples, the one or more images may be stored in the database and retrieved from the database in DICOM format. In some examples, the one or more images may be comparable images. For example, a user may define a body region and all comparable images that include data of the body region. As an example, a user may define abdomen as the body region and comparable images such as abdominal CTs, abdominal MRIs, abdominal PET scans, and the like, may be obtained from the database. Other methods for selecting metric for defining comparable images may be used, for example a reference study may be defined, such as a most recently obtained study, for which other images may be compared to in order to define which studies are comparable.

The obtained one or more images may be time ordered based on acquisition data, as noted at 904. Time ordering the one or more images may define longitudinally adjacent images. Time ordering the one or more images may thus define next and previous images respective to each of the one or more images, as well as defining a current image (e.g., a most recently acquired image) and a reference image (e.g., a first acquired image of the one or more images).

At 906, method 900 includes obtaining annotation data and findings data for each of the one or more images. Data of the one or more images may include annotation data information, including acquisition date, image series, image phase, fusion percentage, various image view types and/or rendering modes, orientation type, and patient orientation for each image view type as well as data of the scan including window width, window level, radiotracer/contrast concentration, display field of view (DFOV), and the like. Further, data of the one or more images may include findings data, including data of one or more objects. As an example, one or more segmentation protocols may be performed when obtaining findings data to define presence and data of the one or more objects within each of the one or more images. The findings data may comprise individual size data, SUV data, and the like for each object within each of the one or more images. For example, the size data may include maximum radius, minimum radius, and the like, as well as comparison size data for each object across longitudinally adjacent images, such as percent change amounts between longitudinally adjacent images.

At 908, method 900 includes determining a configuration of image viewports and findings viewports for a GUI. The GUI may have available preset configurations, such as a grid configuration (e.g., the first configuration of FIG. 2), a horizontal configuration (e.g., the second configuration of FIGS. 3-4), and a comparison configuration (e.g., the third configuration of FIGS. 5-8). The computing device may have a predetermined default configuration stored in memory. In some examples, the predetermined default configuration, such as the grid configuration, may be the configuration that is determined for the GUI. In other examples, a user may define preferences for their profile within the system, including a default configuration preference. If a default configuration preference is defined for the user, the configuration determined at 908 may be the preferred default configuration as defined by the user. In yet further examples, a user may select one of the available configurations in order to determine the configuration.

At 910, method 900 includes displaying the GUI with one or more image viewports and one or more corresponding findings viewports in the determined configuration. As noted above, the determined configuration may be the grid configuration, the horizontal configuration, or the comparison configuration. The one or more images may be displayed within the GUI according to the configuration, as noted at 912, and the image findings for each of the displayed images may be displayed according to the configuration, as noted at 914.

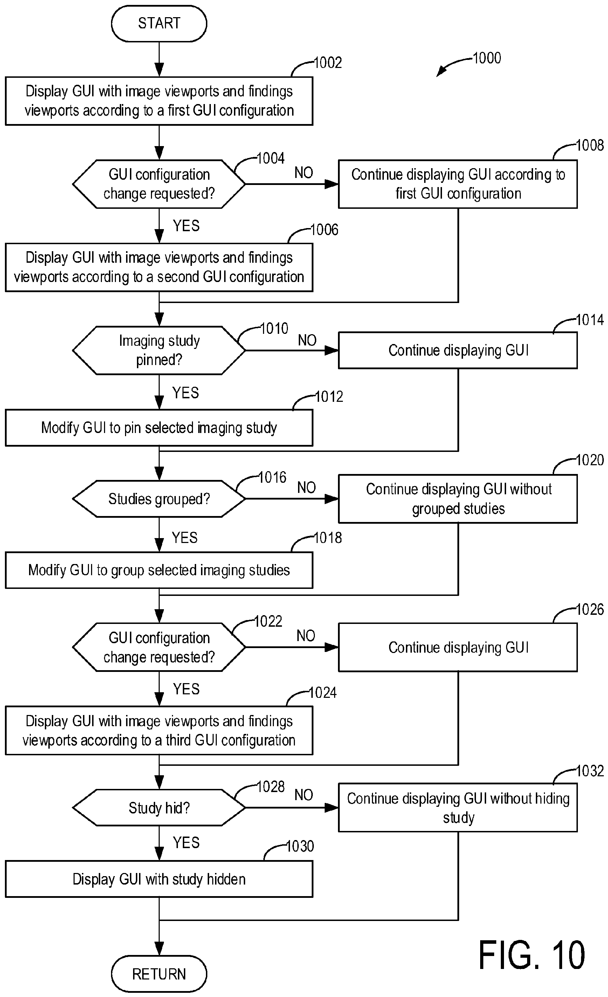

As an example, when the determined configuration is the grid configuration, displaying the one or more images in viewports may comprise displaying an imaging viewport for each of the one or more images in a grid arrangement, whereby images are displayed from left to right in rows based on the time ordering thereof, as described with respect to FIG. 2. In the grid configuration, a findings viewports displaying findings information for a selected image viewport) may be displayed as a side panel to the grid of image viewports. When the determined configuration is the horizontal configuration, image viewports may be displayed in a single row in chronological order from left to right across a first portion of the GUI and corresponding findings viewports may be displayed in a single row across a second portion of the GUI, as described with respect to FIGS. 3-4. The findings viewports may directly correspond to an image viewport and may be aligned in the display as such. When the determined configuration is the comparison configuration, two image viewports may be displayed horizontally adjacent to each other, the images displayed therein may be, as a default, the two most recently acquired images, or alternatively may be user selected images, as described with respect to FIG. 5. In the comparison configuration, findings viewports corresponding to the two image viewports may be displayed horizontally adjacent to each other. The information displayed within a first of the findings viewports may correspond to the image displayed within a first of the image viewports and information displayed within a second of the findings viewports may correspond to the image displayed within a second of the image viewports. As will be further described with respect to FIG. 10, user interactions with the GUI may modify the GUI by way of changing the configuration to one of the other available configurations, pinning a study, grouping studies, and/or hiding/un-hiding studies.

The GUI as herein presented may thus display multiple comparable images acquired across multiple dates within the same window. Thus, the user may visualize objects within multiple images in a comparative manner without having to individually open each image within a PACS application and individually resize each application window. Additionally, the GUI may be configured to automatically size image and findings viewports to fit the GUI window for a given GUI configuration. For example, the grid configuration may display a number of viewports according to the number of obtained comparable images and may proportionally resize the image viewports in response to change in size of the GUI window and/or in response to user interaction that results in closing of the findings viewport.

Turning now to FIG. 10, a flowchart illustrating a method for modifying a displayed multi-image GUI is shown. The method 1000 may be carried out using the systems and components described herein above with regard to FIG. 1. For example, the method 1000 may be carried out according to instructions stored in memory of one or more processors of a computing device, such as computing device 102, that is communicatively coupled to a display device configured to display a GUI, such as first GUI 200, second GUI 300, and/or third GUI 500. It should be appreciated that while the steps of the method 1000 are presented in a particular order, modification of the GUI as herein described may be performed in any reasonable or feasible order.

At 1002, method 1000 includes displaying a GUI with image viewports and findings viewports according to a first GUI configuration. As described above, the GUI may display multiple images acquired at multiple dates and in some instances with multiple modalities within the same window. The first GUI configuration may be a default configuration defined by the system or defined according to user preferences. The first GUI configuration may be one of the grid configuration, the horizontal configuration, and the comparison configuration.

At 1004, method 1000 includes determining whether a first GUI configuration change is requested. A change in GUI configuration may be requested by a user by user selection of an element within the GUI. For example, when the first configuration is the grid configuration, the GUI may comprise a horizontal configuration element and a comparison configuration element. User selection of the comparison configuration element may indicate to the system that a change from the grid configuration to the comparison configuration is requested. If a GUI configuration change is requested, method 1000 proceeds to 1006. If a GUI configuration change is not requested, method 1000 proceeds to 1008 to continue displaying the GUI according to the first GUI configuration. Following 1008, method 1000 may proceed to 1010.

At 1006, method 1000 includes displaying the GUI with image viewports and findings viewports according to a second GUI configuration. The second GUI configuration may the GUI configuration selected within the GUI when in the first configuration that indicated the configuration change was indicated. As an example, the first GUI configuration may be a grid configuration and the second GUI configuration may be a comparison configuration.

At 1010, method 1000 includes determining whether an imaging study is pinned. When 1010 follows 1008, the first GUI configuration may be the comparison configuration. When 1010 follows 1006, the second GUI configuration may be the comparison configuration. An imaging study may be pinned upon user selection of a pin element corresponding to a displayed image. The pin element, as described with respect to FIG. 5, may be included within a heading of a findings viewport of an image. If an imaging study is pinned, method 1000 proceeds to 1012. If an imaging study is not pinned, method 1000 proceeds to 1014 to continue displaying the GUI in the comparison configuration. Following 1014, method 1000 proceeds to 1016.

At 1012, method 1000 includes modifying the GUI to pin the selected imaging study. As described with respect to FIG. 6, pinning a study may pin the image within a given image viewport and the findings thereof within a corresponding findings viewport. When the user alters which images are displayed within the GUI, for example via a displayed image change element, the pinned study may not be removed from display and an image within a second image viewport and findings thereof may be shifted in response to user inputs, thus allowing the user to compare the selected study to different selected studies.

At 1016, method 1000 includes determining whether imaging studies are grouped. Two or more imaging studies may be grouped together based on user selection of grouping elements within a findings viewport of a selected image, as described with respect to FIG. 7. The user may indicate which study or studies are to be grouped with the selected study. If two or more studies are to be grouped, method 1000 proceeds to 1018. If not, method 1000 proceeds to 1020 to continue displaying the GUI without grouped studies. Following 1020, method 1000 proceeds to 1022.

At 1018, method 1000 includes modifying the GUI to group selected imaging studies. As described with respect to FIG. 8, the modified GUI with grouped studies may include multiple grouped image viewports and a grouped findings viewport that indicates findings of one of the grouped images, both of the grouped images individually if different or at the same time if the same, or an average of the grouped images.

At 1020, method 1000 includes determining whether a second GUI configuration change is requested. A change in GUI configuration may be requested by a user by user selection of an element within the GUI. For example, when the second configuration is the comparison configuration, the GUI may comprise a horizontal configuration element and a grid configuration element. User selection of the horizontal configuration element may indicate to the system that a change from the comparison configuration to the horizontal configuration is requested. If a GUI configuration change is requested, method 1000 proceeds to 1024. If a GUI configuration change is not requested, method 1000 proceeds to 1026 to continue displaying the GUI as is (e.g., according to the first or second configuration). Following 1026, method 1000 may proceed to 1028.

At 1024, method 1000 includes displaying the GUI with image viewports and findings viewports according to a third GUI configuration. The third GUI configuration may the GUI configuration selected within the GUI when in the second configuration that indicated the configuration change was indicated. As an example, the second GUI configuration may be a comparison configuration and the third GUI configuration may be a horizontal configuration.

At 1028, method 1000 includes determining whether a study was hidden. When 1028 follows 1026, the first or second GUI configuration may be the horizontal configuration, depending on steps taken previously. When 1028 follows 1024, the third GUI configuration may be the horizontal configuration. A study may be hid in response to user selection of a hide element. The hide element may be displayed within a heading of a findings viewport for a given imaging study and selection of the hide element may indicate that the findings viewport and the image viewport of the given imaging study are to be hidden. If a study was hid, method 1000 proceeds to 1030. If a study was not hid, method 1000 proceeds to 1032 to continue displaying the GUI without hiding a study.

At 1030, method 1000 includes displaying the GUI with the study hidden. Displaying the GUI with the study hidden may include altering display sizes of the remaining image viewports to fill the GUI window with the study hidden.

The modifications and interaction to the GUI as herein described are exemplary in nature. Other actions, including shifting of which images are displayed, toggling between different configurations, interacting with displayed images including image annotations, selecting images, selecting objects within findings viewports, and the like are also provided by the systems herein disclosed.

The technical effect of the systems and methods herein provided is that multiple images are displayed within a single GUI, thereby reducing user interaction (e.g., number of clicks, manual resizing of image windows, and the like) with the system and saving the user time. Further, processing power may be reduced as all comparable studies may be obtained at once, thus reducing the number of times a database is accessed compared to the user needing to open each image they wish to view individually. Further, the GUIs as herein presented may provide information of findings within comparable images, allowing users to easily track evolution of lesions over time. Thus, the system herein described provides a practical application for specific improvement of medical systems by allowing users to view multiple comparable images within the same application, thus reducing the need for users to open each image individually to compare them. As an example, a user may launch the GUI in a horizontal configuration that displays all comparable images and findings thereof in a time ordered, longitudinal configuration, thereby allowing the user to visualize an object within the displayed images over time as well as view measured sizes and/or calculated SUVs of the object over time.

Thus, via the disclosed system herein, comparable images and findings information thereof may be displayed in a manner that is easy to visually parse in a reduced amount of time. The patient information, including the images and findings data may be displayed via small graphical elements with minimal text, which may allow for a larger amount of information to be displayed to the user at once. The data displayed may be stored in various repositories, including PACS, EMRs, and the like, and via the GUIs herein disclosed, the patient information may be aggregated and displayed for easier viewing. This may reduce cognitive overloads and aid clinical thinking, because the patient data is constructed into a clinically helpful structure and demand of the user to open, size configure, and otherwise manipulate the images and data is reduced.

The disclosure also provides support for a computing device comprising a display screen, the computing device being configured to display on the display screen a plurality of image viewports within a multi-image graphical user interface (GUI), the plurality of image viewports displaying respective medical images of a patient, and additionally being configured to display within the GUI one or more findings viewports each displaying findings data corresponding to an image displayed within one of the plurality of image viewports, wherein the findings data of each respective medical image of the patient is obtained from a database while the database is in an un-launched state, and wherein the respective medical images are comparable images of one or more imaging modalities. In a first example of the system, the GUI is displayed on the display screen in one of a grid configuration, a comparison configuration, and a horizontal configuration. In a second example of the system, optionally including the first example in the grid configuration, the plurality of image viewports are arranged in a grid and images are displayed longitudinally according to time order, and the one or more findings viewports comprises a findings viewport corresponding to a selected one of the plurality of image viewports. In a third example of the system, optionally including one or both of the first and second examples in the comparison configuration, the plurality of image viewports comprises a first image viewport and a second image viewport and the one or more findings viewports comprise a first findings viewport that corresponds to the first image viewport and a second findings viewport that corresponds to the second image viewport, wherein findings data of a first image that is displayed within the first image viewport is displayed within the first findings viewport and findings data of a second image that is displayed within the second image viewport is displayed within the second findings viewport. In a fourth example of the system, optionally including one or more or each of the first through third examples in the horizontal configuration, the plurality of image viewports are arranged side by side along a first portion of the GUI and the one or more findings viewports are arranged side by side along a second portion of the GUI, wherein each of the findings viewports corresponds to and vertically aligns with one of the plurality of image viewports to display findings data of an image displayed within a corresponding image viewport. In a fifth example of the system, optionally including one or more or each of the first through fourth examples, the GUI comprises one or more selectable elements configured to toggle between the grid configuration, comparison configuration, and horizontal configuration. In a sixth example of the system, optionally including one or more or each of the first through fifth examples when in the comparison configuration, the GUI comprises a pin element that when selected for a given displayed image, pins the given displayed image to the GUI, and a grouping element that when selected for a displayed image triggers display of a pop-up menu listing available images with which to group the selected displayed image. In a seventh example of the system, optionally including one or more or each of the first through sixth examples, each findings viewport comprises one or more findings elements, each of the one or more findings elements displaying information of an object for a corresponding medical image.

The disclosure also provides support for a method for displaying multiple images in a graphical user interface (GUI), comprising: obtaining one or more medical images and findings data thereof from a database, time ordering the obtained one or more medical images and findings data thereof, and displaying the one or more medical images in respective image viewports and findings data of at least one of the one or more medical images in at least one findings viewport of the GUI, wherein display of the respective image viewports and the at least one findings viewport is based on a selected GUI configuration, wherein the one or more medical images are comparable images of one or more imaging modalities. In a first example of the method, the image viewports comprise a first image viewport corresponding to a first findings viewport and a second image viewport corresponding to a second findings viewport, wherein the first image viewport and the first findings viewport correspond to a first color and the second image viewport and the second findings viewport correspond to a second, different color. In a second example of the method, optionally including the first example, the method further comprises:, when the selected GUI configuration is one of the grid configuration and the comparison configuration, modifying findings data displayed within the at least one findings viewport in response to user selection of one of a next element and a previous element, wherein the next element triggers display of data corresponding to a next later acquired image and the previous element triggers display of data corresponding to a next earlier acquired image. In a third example of the method, optionally including one or both of the first and second examples, each of the respective image viewports are selectable and, when the selected GUI configuration is one of the grid configuration and the comparison configuration, selection of one of the next element and the previous element selects a corresponding image viewport that displays one of the next later acquired image and the next earlier acquired image, respectively. In a fourth example of the method, optionally including one or more or each of the first through third examples, the method further comprises:, when the selected GUI configuration is the comparison configuration, pinning an image to the GUI in response to user selection of a pin element corresponding to the image, wherein, when one or both of the next element and the previous element are selected when the image is pinned, the pinned image is not removed from the display. In a fifth example of the method, optionally including one or more or each of the first through fourth examples, the method further comprises:, when the selected GUI configuration is the horizontal configuration, removing an image viewport and a corresponding findings viewport from the GUI in response to user selection of a hide element corresponding to the corresponding findings viewport. In a sixth example of the method, optionally including one or more or each of the first through fifth examples, each findings viewport comprises one or more findings elements each corresponding to an object identified within one or more of the one or more medical images. In a seventh example of the method, optionally including one or more or each of the first through sixth examples, the selected GUI configuration is one of a predefined default configuration, a user preference default configuration, or a configuration selected via user input.

The disclosure also provides support for a system, comprising: one or more imaging systems configured to acquire medical imaging data of a patient, wherein the one or more imaging systems are coupled to a database configured to store the medical imaging data and to a computing device configured with instructions stored in non-transitory memory executable by a processor that, when executed, cause the processor to: display a graphical user interface (GUI) comprising one or more image viewports and one or more findings viewports, wherein each image viewport displays a medical image of the medical imaging data and each findings viewport displays findings data corresponding to a respective medical image displayed within one of the one or more image viewports, wherein the one or more image viewports and the one or more findings viewports are arranged within the GUI according to a first GUI configuration, display one or more findings elements within each of the one or more findings viewports, wherein each of the one or more findings elements corresponds to an object of the medical imaging data, wherein the one or more findings elements display information relating to the object including percent change in volume of the object, and modify the GUI in response to user input to change from the first GUI configuration to a second GUI configuration, wherein in the first GUI configuration a set of comparable images are displayed in respective image viewports and in the second GUI configuration a subset of the set of comparable images are displayed in respective image viewports. In a first example of the system, each of the one or more findings elements are selectable elements that, when selected, highlight the corresponding object within each respective displayed medical image. In a second example of the system, optionally including the first example, user selection displaying an image slice within a selected image viewport triggers display of comparable image slices within each of the other image viewports. In a third example of the system, optionally including one or both of the first and second examples, each of the one or more findings elements comprise one or more metrics, including percent change of the one or more metrics compared to a reference image.

As used herein, an element or step recited in the singular and proceeded with the word "a" or "an" should be understood as not excluding plural of said elements or steps, unless such exclusion is explicitly stated. Furthermore, references to "one embodiment" of the present invention are not intended to be interpreted as excluding the existence of additional embodiments that also incorporate the recited features. Moreover, unless explicitly stated to the contrary, embodiments "comprising," "including," or "having" an element or a plurality of elements having a particular property may include additional such elements not having that property. The terms "including" and "in which" are used as the plain-language equivalents of the respective terms "comprising" and "wherein." Moreover, the terms "first," "second," and "third," etc. are used merely as labels, and are not intended to impose numerical requirements or a particular positional order on their objects.

This written description uses examples to disclose the invention, including the best mode, and also to enable a person of ordinary skill in the relevant art to practice the invention, including making and using any devices or systems and performing any incorporated methods. The patentable scope of the invention is defined by the claims, and may include other examples that occur to those of ordinary skill in the art. Such other examples are intended to be within the scope of the claims if they have structural elements that do not differ from the literal language of the claims, or if they include equivalent structural elements with insubstantial differences from the literal languages of the claims.

## Claims

1. A computing device (102) comprising a display screen (134), the computing device being configured to display on the display screen a plurality of image viewports (204) within a multi-image graphical user interface (GUI) (200), the plurality of image viewports displaying respective medical images of a patient, and additionally being configured to display within the GUI one or more findings viewports (206) each displaying findings data corresponding to an image displayed within one of the plurality of image viewports, wherein the findings data of each respective medical image of the patient is obtained from a database (150) while the database is in an un-launched state, and wherein the respective medical images are comparable images of one or more imaging modalities.

2. The computing device of claim 1, wherein the GUI (200) is displayed on the display screen (134) in one of a grid configuration, a comparison configuration, and a horizontal configuration.

3. The computing device of claim 2, wherein, in the grid configuration, the plurality of image viewports (204) are arranged in a grid and images are displayed longitudinally according to time order, and the one or more findings viewports (206) comprises a findings viewport corresponding to a selected one of the plurality of image viewports.

4. The computing device of claim 2, wherein, in the comparison configuration, the plurality of image viewports (504) comprises a first image viewport (506) and a second image viewport (508) and the one or more findings viewports (510) comprise a first findings viewport (512) that corresponds to the first image viewport and a second findings viewport (514) that corresponds to the second image viewport, wherein findings data of a first image that is displayed within the first image viewport is displayed within the first findings viewport and findings data of a second image that is displayed within the second image viewport is displayed within the second findings viewport.

5. The computing device of claim 2, wherein, in the horizontal configuration, the plurality of image viewports (304) are arranged side by side along a first portion of the GUI (300) and the one or more findings viewports (306) are arranged side by side along a second portion of the GUI, wherein each of the findings viewports corresponds to and vertically aligns with one of the plurality of image viewports to display findings data of an image displayed within a corresponding image viewport.

6. The computing device of claim 2, wherein the GUI (200, 300, 500) comprises one or more selectable elements (252, 254) configured to toggle between the grid configuration, comparison configuration, and horizontal configuration.

7. The computing device of claim 2, wherein, when in the comparison configuration, the GUI (500) comprises a pin element (522) that when selected for a given displayed image, pins the given displayed image to the GUI, and a grouping element (706) that when selected for a displayed image triggers display of a pop-up menu listing available images with which to group the selected displayed image.

8. The computing device of claim 1, wherein each findings viewport comprises one or more findings elements (524), each of the one or more findings elements displaying information of an object (246) for a corresponding medical image.

9. A method for displaying multiple images in a graphical user interface (GUI), comprising:
obtaining one or more medical images and findings data thereof from a database (902);
time ordering the obtained one or more medical images and findings data thereof (904); and
displaying the one or more medical images in respective image viewports and findings data of at least one of the one or more medical images in at least one findings viewport of the GUI (910), wherein display of the respective image viewports and the at least one findings viewport is based on a selected GUI configuration (908), wherein the one or more medical images are comparable images of one or more imaging modalities.

10. The method of claim 9, wherein the image viewports comprise a first image viewport (506) corresponding to a first findings viewport (512) and a second image viewport (508) corresponding to a second findings viewport (514), wherein the first image viewport and the first findings viewport correspond to a first color and the second image viewport and the second findings viewport correspond to a second, different color.

11. The method of claim 10, further comprising, when the selected GUI configuration is one of the grid configuration and the comparison configuration, modifying findings data displayed within the at least one findings viewport in response to user selection of one of a next element (234) and a previous element (236), wherein the next element triggers display of data corresponding to a next later acquired image and the previous element triggers display of data corresponding to a next earlier acquired image.

12. The method of claim 11, wherein each of the respective image viewports are selectable and, when the selected GUI configuration is one of the grid configuration and the comparison configuration, selection of one of the next element (234) and the previous element (236) selects a corresponding image viewport that displays one of the next later acquired image and the next earlier acquired image, respectively.

13. The method of claim 11, further comprising, when the selected GUI configuration is the comparison configuration, pinning an image to the GUI in response to user selection of a pin element (522) corresponding to the image, wherein, when one or both of the next element (234) and the previous element (236) are selected when the image is pinned, the pinned image is not removed from the display.

14. The method of claim 10, further comprising, when the selected GUI configuration is the horizontal configuration, removing an image viewport and a corresponding findings viewport from the GUI in response to user selection of a hide element (322) corresponding to the corresponding findings viewport.

15. The method of claim 9, wherein each findings viewport comprises one or more findings elements each corresponding to an object (246) identified within one or more of the one or more medical images.
